# EUROPÄISCHE PATENTSCHRIFT

(11) **EP 1 370 178 B1**
(45) Veröffentlichungstag und Bekanntmachung des Hinweises auf die Patenterteilung: **24.08.2005**
(21) Anmeldenummer: 02721996.3
(22) Anmeldetag: 07.03.2002
(51) Int. Cl.: A61B 6/04

(54) **MEDIZINISCHE UNTERSUCHUNGS- UND/ODER BEHANDLUNGSEINRICHTUNG MIT EINEM TRANSPORTWAGEN FÜR EINE PATIENTENLIEGE UND TRANSPORTWAGEN**
MEDICAL EXAMINATION AND/OR TREATMENT DEVICE WITH A TRANSPORT CARRIAGE FOR A PATIENT BED AND TRANSPORT CARRIAGE
DISPOSITIF D'EXAMEN ET/OU DE TRAITEMENT MEDICAL COMPRENANT UN CHARIOT TRANSPORTEUR POUR UN LIT D'EXAMEN ET CHARIOT TRANSPORTEUR

(30) Priorität: 21.03.2001 DE 10113855
(43) Veröffentlichungstag der Anmeldung: 17.12.2003
(73) Patentinhaber: SIEMENS AKTIENGESELLSCHAFT, 80333 München (DE)
(72) Erfinder: BARTELS, Frank, 95466 Weidenberg (DE); HEINOLD, Michael, Clayton, CA 94517 (US); REIMANN, Rolf, 91301 Forchheim (DE)
(86) Internationale Anmeldenummer: PCT/DE2002/000824
(87) Internationale Veröffentlichungsnummer: WO 2002/076298

(56) Entgegenhaltungen:
- DE-A- 3 034 932
- GB-A- 961 677
- US-A- 3 917 076
- US-A- 5 842 987
- US-A- 6 101 644
- US-B1- 6 205 347

## Beschreibung

Die Erfindung betrifft einen Transportwagen für eine Patientenliege, insbesondere für eine Einrichtung zur medizinischen Untersuchung und/oder Behandlung eines Patienten, mit einem Fahrgestell, und mit Auslegerarmen zur Aufnahme der Patientenliege.

Die genannte Einrichtung zur medizinischen Untersuchung und/oder Behandlung eines Patienten umfasst mehrere Untersuchungs- und/oder Behandlungsgeräte unterschiedlichen Typs, insbesondere mit einem Kernspintomographen und/oder einem Computertomographen und/oder einem Angiographiegerät und/oder einem Nukleartherapiegerät, wobei die Untersuchungs- und/oder Behandlungsgeräte jeweils eine Patientenlagerungsvorrichtung mit einer Patientenliege aufweisen, wobei insbesondere die jeweilige Patientenliege in einen Untersuchungs- bzw. Behandlungsbereich des jeweiligen Untersuchungs- und/oder Behandlungsgeräts einführbar ist, und wenigstens einen Transportwagen.

Transportwägen, die die Übergabe eines Patienten an ein Tischgestell eines Untersuchungsgeräts ermöglichen, sind z.B. bekannt aus der DE 30 34 932 A1 und der DE 42 24 036 C1.

Aus der US 6,205,347 A ist eine medizintechnische Einrichtung mit mehreren Untersuchungsgeräten bekannt, nämlich einem Computertomographen und einem nuklearmedizinischen bildgebenden Gerät. Ein Patient kann mittels einer schwenkbaren Patientenliege zu den einzelnen Untersuchungsgeräten transportiert werden. Eine einzelne, fahrbare Patientenliege ist beispielsweise aus der US 6,101,644 A bekannt. In diesem Fall ist ein Liegenbrett von einem fahrbaren Untergestell abnehmbar.

In einem großen Krankenhaus sind in der Regel mehrere voneinander verschiedene medizinische Untersuchungsgeräte und/oder mehrere verschiedene Therapiegeräte vorhanden. Jedes Untersuchungs- und/oder Behandlungsgerät weist eine spezielle Patientenlagerungsvorrichtung mit einer Patientenliege auf, die positionsstarr oder beweglich auf einem Sockel angebracht ist. Auf der Patientenliege ruht der Patient während der Untersuchung bzw. Behandlung. Um den Patienten in den Untersuchungs- bzw. Behandlungsbereich des jeweiligen Untersuchungs- und/oder Behandlungsgeräts einzuführen, beispielsweise in die Röhre des Kernspintomographen, ist die Patientenliege insbesondere auf dem Sockel beweglich gelagert oder geführt.

Zum Transport zu oder von einem Untersuchungs- und/oder Behandlungsgerät wird ein Transportwagen benutzt, der auch als Trolley bezeichnet wird. Der Patient wird hierzu auf dem Transportwagen gelagert oder gebettet. Dabei ist jeweils ein Umbettungs- oder Umlagerungsvorgang notwendig, der für das medizinische Personal zeitraubend und körperlich anstrengend ist. Außerdem könnten stark geschädigte Patienten, beispielsweise Trauma-Patienten, bei einem Umbettungsvorgang weitere Schäden erleiden. Zudem geht beim Umbetten wertvolle Zeit verloren.

Der Erfindung liegt die Aufgabe zugrunde, einen Transportwagen anzugeben, bei dem diese Nachteile vermieden oder zumindest gemildert sind. Diese Aufgabe wird durch einen Transportwagen mit den Merkmalen des Anspruchs 1 gelöst. Die Patientenliegen sind von der jeweiligen Patientenlagerungsvorrichtung abnehmbar. Die Patientenliege und der Transportwagen sind derart ausgebildet, das jede der Patientenliegen an den Transportwagen ankoppelbar ist.

Daraus ergibt sich der Vorteil, dass der Patient beim Fahren zu oder von einem Untersuchungs- und/oder Behandlungsgerät bereits auf der für das jeweilige Untersuchungs- und/oder Behandlungsgerät und die zugeordnete Patientenlagerungsvorrichtung notwendigen Patientenliege gelagert sein kann. Dadurch ist die Anzahl der notwendigen Umlagerungs- oder Umbettungsvorgänge in für das Krankenhauspersonal vorteilhafter Weise reduziert. Das Krankenhauspersonal kann ergonomisch günstiger und weniger anstrengend arbeiten. Dadurch, dass jede der Patientenliegen an den Transportwagen ankoppelbar ist, ist dieser Vorteil erreichbar, ohne dass hierzu eine besonders hohe Anzahl unterschiedlicher Transportwägen erforderlich wäre. Anders formuliert bedeutet dies, dass die Wahrscheinlichkeit, dass das Krankenhauspersonal an einem bestimmten Ort oder Untersuchungs- bzw. Behandlungsraum im Krankenhaus gerade einen passenden Transportwagen vorfindet, hoch ist, auch bei einer nur geringen Anzahl insgesamt dort vorhandener Transportwagen.

Die Patientenliegen sind an den Transportwagen insbesondere fest ankoppelbar, d.h., dass im angekoppelten Zustand ein Abheben und Verschieben der Patientenliege auf dem Transportwagen unterbunden ist. Im gekoppelten Zustand ist eine Bewegung in alle sechs Raumrichtungen unterbunden. Die Kopplung kann kraftschlüssig und/oder formschlüssig geschehen.

Außerdem können die Patientenliegen zumindest an die Patientenlagerungsvorrichtung ihres jeweiligen Untersuchungs- und/oder Behandlungsgeräts ankoppelbar sein.

Nach einer besonders bevorzugten Ausgestaltung sind die Patientenliegen und der Transportwagen derart ausgebildet, dass die Patientenliegen an einem Ende des Transportwagens auf diesen auflegbar und am gegenüberliegenden Ende an diesen ankoppelbar sind. Bei dieser Ausführungsform wird also eine feste Verbindung zwischen den Patientenliegen und dem Transportwagen nur an einem Ende des Transportwagens hergestellt. Daraus ergeben sich mehrere Vorteile. Zum einen müssen die Patientenliegen an dem Ende, an welchem sie auf den Transportwagen auflegbar sind, nicht mit einem Koppelmechanismus versehen werden, der möglicherweise metallisch gefertigt werden müsste. Dadurch ist gewährleistet, dass dieses Ende der Patientenliegen beispielsweise in die Öffnung eines Kernspintomographen oder eines Computertomographen einführbar ist, ohne deren Betrieb zu stören (Magnetfeld bzw. Röntgenabsorption o.dgl.). Ein weiterer Vorteil besteht darin, das - ausgehend von einem bekannten Untersuchungs- und/oder Behandlungsgerät mit einer fest zugeordneten Patientenliege - die Patientenliege an der einen Seite nicht, zumindest nicht wesentlich, verändert werden muss. Dies ist von Bedeutung für eine Nachrüstung bereits bestehender medizinischer Untersuchungs- und/oder Behandlungseinrichtungen. Es besteht weiterhin der Vorteil, dass bei einem Auflegen der Patientenliege an dem einen Ende des Transportwagens der Transportwagen derart ausgestaltbar ist, dass eine Behinderung des Krankenhauspersonals vermieden ist, d.h. der Patient ist optimal zugänglich.

Vorzugsweise sind die Patientenliegen und der Transportwagen patientenfußseitig koppelbar. Ebenfalls bevorzugt sind die Patientenliegen patientenkopfseitig auf den Transportwagen auflegbar.

Nach einer bevorzugten Weiterbildung weisen die Patientenliegen identische Koppeleinrichtungen zum lösbaren Befestigen am Transportwagen auf. Diese Koppeleinrichtungen können außerdem zur Ankopplung an die Patientenlagerungsvorrichtungen der Untersuchungs- und/oder Behandlungsgeräte ausgebildet sein.

Hierzu weist der Transportwagen bevorzugt ein Koppelmittel zur Aufnahme der Koppeleinrichtung der Patientenliegen auf, wobei durch Betätigung des Koppelmittels die jeweilige Patientenliege vom Transportwagen lösbar ist. Bei dieser Variante ergibt sich der Vorteil, dass der größere Teil des Koppelmechanismus am Transportwagen angebracht sein kann und dass die - gegebenenfalls bereits vorhandenen - Patientenliegen nur geringfügig modifiziert werden müssen.

Ein erster Auslegerarm der Auslegerarme des Transportwagens ist zum nicht koppelnden Auflegen der Patientenliege und ein zweiter Auslegerarm der Auslegerarme zum Ankoppeln der Patientenliege ausgebildet.

Ein solcher Transportwagen ist in besonders einfacher Weise zur universellen Verwendbarkeit für mehrere medizinische Untersuchungs- und/oder Behandlungsgeräte ausbildbar. Die auf dem ersten Auslegerarm zu liegen kommende Seite der Patientenliege muss entweder gar nicht oder nur geringfügig an ihrer Unterseite modifiziert werden. Ein Koppelmechanismus, der - weil aus Metall gefertigt - die Bildgebung in einem Röntgen-, Computertomographie- oder Kernspintomographiegerät stören könnte, ist nicht nötig.

Außerdem bleibt der Patient an dieser Seite der Patientenliege uneingeschränkt zugänglich. Auch aus diesem Grunde ist der erste Auslegerarm vorzugsweise patientenkopfseitig angebracht, d.h. der kopfseitige Teil der Patientenliege kommt dort zur Auflage.

Der zweite Auslegerarm ist derart ausgebildet, dass die Patientenliege im angekoppelten Zustand unterhalb des zweiten Auslegerarmes zu liegen kommt. Auf diese Weise ist es vorteilig möglich, eine bereits vorhandene Patientenliege in einfacher Weise nachzurüsten, ohne an ihrer Unterseite, die in der Regel an die jeweilige Patientenlagerungsvorrichtung spezifisch angepasst ist, einzugreifen.

Insbesondere ist der zweite Auslegerarm zur hängenden Aufnahme der Patientenliege ausgebildet. Es sind aber auch Varianten möglich, bei denen der zweite Auslegerarm die Patientenliege, deren Oberseite unter- oder umgreifend, hält.

Insbesondere bei der hängenden Aufnahme der Patientenliege ist es zweckmäßig, dass der zweite Auslegerarm ein Koppelmittel zur Herstellung einer festen, lösbaren Verbindung mit der Patientenliege aufweist. Ein solches Koppelmittel ist in einfacher Weise z.B. durch einen Rast- oder Schnappmechanismus realisierbar.

Das Ankoppeln des zweiten Auslegerarmes an die Patientenliege, insbesondere mit dem Koppelmittel, kann durch Kraft- und/oder Formschluss geschehen, insbesondere derart, dass im gekoppelten Zustand die Patientenliege weder zur Seite noch nach oben oder unten bewegbar ist.

Dagegen kann bei dem Transportwagen nach der Erfindung eine solche starre Verbindung zur Patientenliege bei dem ersten Auslegerarm unterbleiben. Der erste Auslegerarm ist vorzugsweise derart ausgebildet, dass die aufliegende Patientenliege gegen seitliches Verschieben gesichert ist, vorzugsweise derart, dass die Patientenliege nach oben beweglich bleibt. Daraus ergibt sich der Vorteil, dass das Anbringen der Patientenliege auf dem Transportwagen für das Krankenhauspersonal vereinfacht ist. Ferner ergibt sich als Vorteil, dass an der Patientenliege kopfseitig, d.h. an der Seite des ersten Auslegerarms, eine Koppeleinrichtung nicht zwingend erforderlich ist, welche - insbesondere falls aus Metall gefertigt - die Bildgebung in einer Untersuchungseinrichtung stören könnte.

Vorzugsweise weist der erste Auslegerarm eine Ausnehmung für die Patientenliege auf, deren Unterseite hierzu insbesondere mit einer Anformung ausgestattet ist, die formschlüssig in die Ausnehmung eingepasst ist und dort eventuell einrastet.

Eine besonders bevorzugte Weiterbildung sieht vor, dass die beiden Auslegerarme jeweils endseitig an einer Teleskopsäule befestigt sind. Dadurch ist es in vorteilhafter Weise möglich, den Transportwagen bei der Übergabe an oder von einem der Untersuchungs- und/oder Behandlungsgeräte an die jeweilige Lagerungshöhe des betreffenden Untersuchungs- und/oder Behandlungsgeräts anzupassen. Beim bevorzugten Einsatz des Transportwagens nach der Erfindung für einen Verbund aus mehreren Untersuchungs- und/oder Behandlungsgeräten unterschiedlichen Typs ist nämlich mit unterschiedlichen Gerätehöhen zu rechnen.

Für die Teleskopsäulen ist es vorteilig, dass diese außermittig auf dem Fahrgestell, insbesondere randseitig auf dem Fahrgestell, angebracht sind. Das Traggestell eines solchen Transportwagens, bestehend u.a. aus dem Fahrgestell, den Teleskopsäulen und den Tragarmen, hat in einer Querschnittsansicht insbesondere die Form eines einseitig offenen Rechtecks oder eines "C". Mit einem solchen Transportwagen ist es besonders vorteilhaft möglich, die Patientenliege an ein Untersuchungs- und/oder Behandlungsgerät zu übergeben oder von diesem abzuholen, ohne dass eine maßgebliche seitliche Verschiebung der Patientenliege notwendig ist. Mit einem solchen Transportwagen ist es vielmehr in besonders vorteilhafter Weise möglich, bei einer Übergabe der Patientenliege vom Transportwagen an ein Untersuchungs- und/oder Behandlungsgerät, die Patientenliege exakt an einer solchen Stelle über einem Sockel der Patientenlagerungsvorrichtung zu positionieren, an welcher die Patientenliege mit dem Unterbau oder Sockel der Patientenlagerungsvorrichtung koppelbar ist. Hierbei kann das Traggestell des Transportwagens stationäre Teile der jeweiligen Patientenlagerungsvorrichtung umschließen. Diese Teile sind dann im Inneren des genannten offenen Rechtecks bzw. "C" positioniert.

Nach einer weiteren bevorzugten Ausgestaltung sind die Teleskopsäulen sowohl gleichlaufend als auch unabhängig voneinander in der Höhe verstellbar. Ein derart ausgestalteter Transportwagen hat den besonderen Vorteil, dass er Patientenliegen unterschiedlicher Dicke oder Höhe aufnehmen kann. Dies ist insbesondere von Bedeutung, falls bei angekoppelter Patientenliege der erste Auslegerarm unterhalb der Patientenliege und der zweite Auslegerarm oberhalb der Patientenliege angeordnet ist.

Vorzugsweise sind die Auslegerarme vom Fahrgestell abnehmbar und gegeneinander austauschbar. Dadurch, dass z.B. der hintere Auslegerarm an der vorderen Position anbringbar und der vordere Auslegerarm an der hinteren Position anbringbar ist, ergibt sich der Vorteil, dass der Transportwagen wahlweise von links oder von rechts an eine Patientenlagerungsvorrichtung anfahrbar ist, wobei in jedem Fall gewährleistet ist, dass der erste Auslegerarm dem Untersuchungsbereich eines Untersuchungsgeräts, beispielsweise der Öffnung eines Kernspintomographen, zuwendbar ist.

Ein Ausführungsbeispiel eines Transportwagens nach der Erfindung und seine Verwendung werden nachfolgend anhand der Figuren 1 bis 5 näher erläutert. Es zeigen:
- Figur 1: einen Transportwagen nach der Erfindung in einer perspektivischen Darstellung,
- Figur 2: ein Detail des Transportwagens der Figur 1,
- Figur 3: den Transportwagen der Figur 1 im Einsatz bei einem Computertomographen,
- Figur 4: den Transportwagen der Figur 1 beim Einsatz bei einem Kernspintomographen, und
- Figur 5: den Transportwagen der Figur 1 in einer weiteren Verwendung.

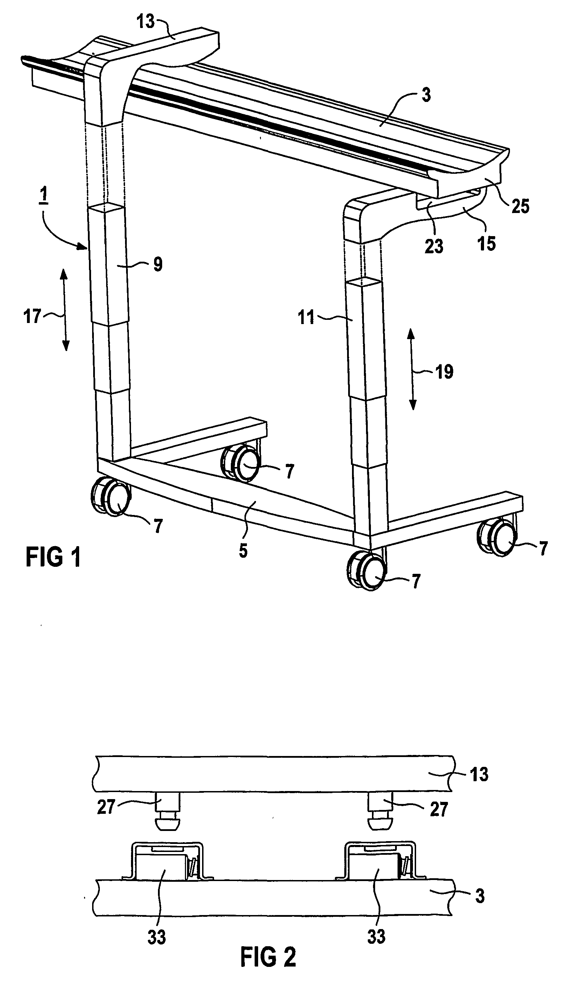

Figur 1 zeigt einen Trolley oder einen Transportwagen 1 zur Aufnahme eines Liegenbretts, einer Traumaplatte, einer Patientenlagerungsplatte oder einer Patientenliege 3. Der Transportwagen 1 weist unten ein U-förmiges oder C-förmiges Fahrgestell 5 auf, an dem vier Doppelrollen 7 angebracht sind. Jeweils zwei der Doppelrollen 7 sind durch Betätigung eines nicht explizit dargestellten Fußtasters verriegelbar.

Auf der geschlossenen Seite des Fahrgestells 5 sind randseitig Teleskopsäulen 9, 11 angebracht, die entweder mittels Muskelkraft oder mittels eines nicht explizit dargestellten Antriebsmotors sowohl gleichlaufend als auch unabhängig voneinander in ihrer Höhe verstellbar sind. Jede der Teleskopsäulen 9, 11 trägt einen horizontal verlaufenden Auslegerarm 13 bzw. 15. Die Auslegerarme 13, 15 dienen der Aufnahme der Patientenliege 3, wobei der erste Auslegerarm 15 die Patientenliege 3 unterstützt und der zweite Auslegerarm 13 von oben an sie angreift. Dadurch, dass die Teleskopsäulen 9, 11 entlang der vertikalen Richtungen 17 bzw. 19 unabhängig voneinander ausfahrbar sind, sind mit dem Transportwagen 1 Patientenliegen 3 unterschiedlicher Dicke transportierbar, obgleich der zweite Auslegerarm von oben und der erste Auslegerarm 15 von unten an die Patientenliege 3 angreifen.

Der erste Auslegerarm 15 ist zum Auflegen der Patientenliege 3 ausgebildet und weist hierzu an seiner Oberseite eine Mulde oder eine breite Ausnehmung 23 auf, welche eine entsprechende Anformung 25 an der Unterseite der Patientenliege derart aufnimmt, dass die Patientenliege 3 an der Stelle des ersten Auslegerarms 15 gegen seitliches Verschieben gesichert ist. Der Transportwagen 1 und die Patientenliege 3 sind derart ausgebildet, dass der Kopf eines Patienten beim ersten Auslegerarm 15 und seine Füße beim zweiten Auslegerarm 13 zu liegen kommen.

Der erste Auslegerarm 15 und der zweite Auslegerarm 13 sind mittels identischer (nicht explizit dargestellter) Steckverbindungen am Fahrgestell 5 befestigt, so dass sie gegeneinander austauschbar sind. Zur Arretierung sind z.B. Sicherungsstifte vorhanden.

Der zweite Auslegerarm 13 ist zum festen Ankoppeln der Patientenliege 3 ausgebildet. Ein möglicher Koppelmechanismus ist in **Figur 2** näher dargestellt. Der zweite Auslegerarm 13 weist Koppelmittel 27 auf, die mit entsprechenden Koppeleinrichtungen 33 an der Oberseite der Patientenliege 3 positionsstarr und lösbar koppelbar sind. Im dargestellten Beispiel sind die Koppelmittel 27 als an ihrer Spitze konusförmig ausgebildete Zapfen mit einer dahinter angeordneten Ringnut ausgebildet. Diese Zapfen sind in die jeweilige Koppeleinrichtung 33 als Gegenstück einrastbar, in dem ein federgelagerter Stift in die Ringnut eingreift.

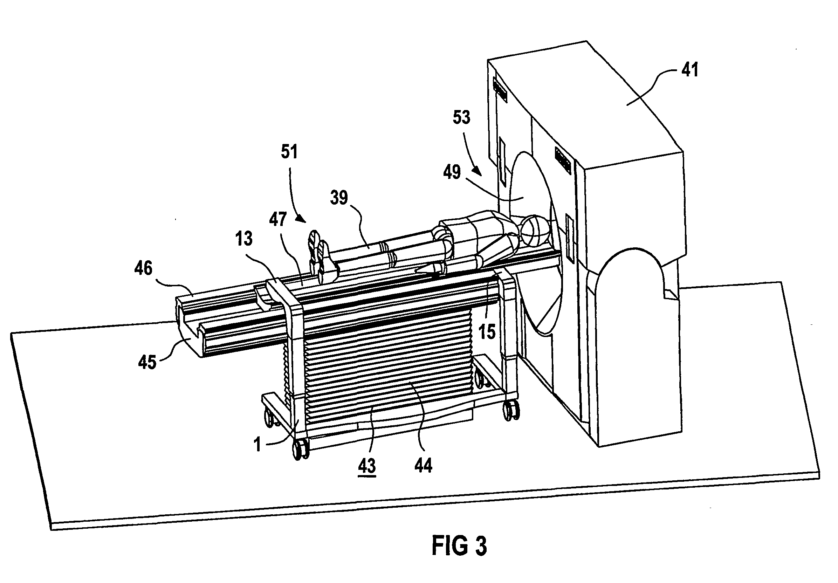

In den Figuren 3, 4 und 5 ist die Verwendung des Transportwagens 1 nach der Erfindung bei unterschiedlichen medizinischen Untersuchungsgeräten schematisch dargestellt.

**Figur 3** zeigt einen Patienten 39 vor der Untersuchung in einem Computertomographen 41. Zu dem Computertomographen 41 gehört eine Patientenlagerungsvorrichtung 43, welche einen Sockel 44 mit einem darauf angeordneten länglichen überstehenden Oberteil 45 umfasst. In dem Oberteil 45 ist ein Schlitten 46 in Längsrichtung verfahrbar. Der Schlitten 46 ist zur Aufnahme einer Patientenliege 47 ausgebildet, so dass die Patientenliege 47 mit aufliegendem Patienten 39 in eine Öffnung 49 im Gehäuse des Computertomographen 41 einführbar ist. Durch Bewegung des Patienten 39 durch die Öffnung 49 findet ein Scan über mehrere Schnittbilder statt. Die Patientenliege kann vom medizinischen Personal in einfacher Weise abgenommen werden, vorzugsweise durch leichtes Anheben.

Der Schlitten kann selbst auch als Lagerungsplatte oder Liege ausgebildet sein.

In Figur 3 ist der Transportwagen 1 mit der Patientenliege 47 unmittelbar vor oder nach einer Übergabe von bzw. an die Patientenlagerungsvorrichtung 43 dargestellt. Um in diese Position zu gelangen, wurde der Patient 39 mittels des Transportwagens 1 zuvor in den Untersuchungsraum mit dem Computertomographen 41 gefahren. Der Transportwagen 1 wurde dann unterhalb des Oberteils 45 und den Sockel 44 einschließend an der Patientenlagerungsvorrichtung 43 positioniert. Dabei wurde der erste Auslegerarm 15 am Kopfende der Patientenlagerungsvorrichtung 43 positioniert, so dass der Patient 39 mit dem Kopf voran in die Öffnung 49 einführbar ist. Der erste Auslegerarm 15 ist dabei zwischen dem gehäuseseitigen Ende der Patientenlagerungsvorrichtung 43 und dem Gehäuse des Computertomographen 41 angeordnet und unterstützt die Patientenliege 47. Am Fußende 51 hängt die Patientenliege 47 zu diesem Zeitpunkt noch am zweiten Auslegerarm 13. Die beiden Teleskopsäulen 9, 11 können in diesem Zeitpunkt in ihrer Höhe unterschiedlich weit ausgefahren sein, falls die Dicke der Patientenliege 47 (= Abstand Oberkante zur Unterkante) dies erfordert.

Zur Übergabe der Patientenliege 47 an die Patientenlagerungsvorrichtung 43 werden die Teleskopsäulen 9, 11 dann simultan eingefahren, bis die Patientenliege 47 auf dem Schlitten 46 zu liegen kommt und daran gegebenenfalls angekoppelt werden kann.

Nach Übergabe der Patientenliege 47 an die Patientenlagerungsvorrichtung 43 wird die Teleskopsäule 9 des zweiten Auslegerarms nach oben und die Teleskopsäule 11 des ersten Auslegerarms 15 nach unten bewegt, so dass der Transportwagen 1 von der Patientenlagerungsvorrichtung 43 weggefahren werden kann. Alternativ hierzu wird die Patientenlagerungsvorrichtung 43 mit ihrer Oberkante nach unten bewegt.

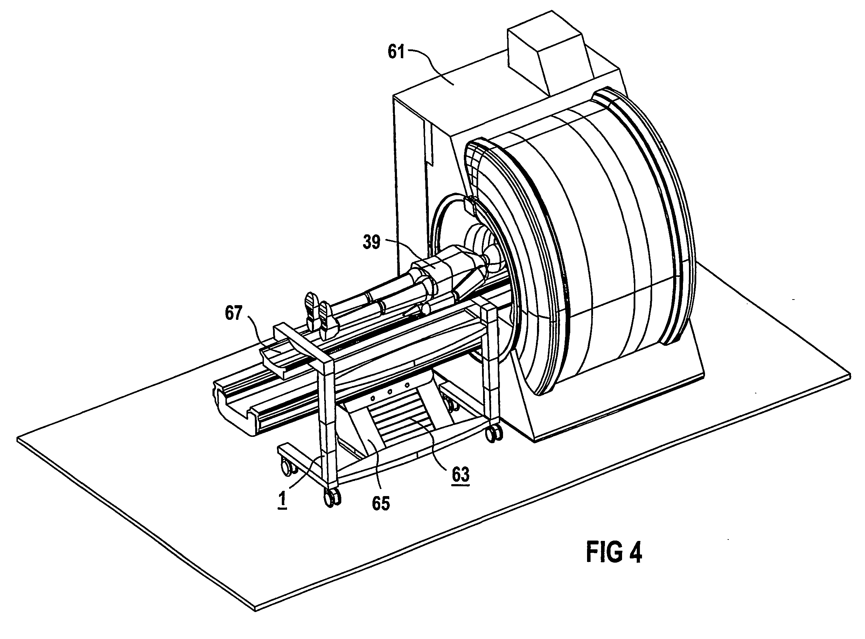

**Figur 4** zeigt den Patienten 39 vor einer Untersuchung in einem Kernspintomographen 61 mit einer Patientenlagerungsvorrichtung 63, die eine Patientenliege 67 aufweist. Die Patientenliege 67 ist - wie alle Patientenliegen der erfindungsgemäßen Einrichtung "(Verbund") - vom Personal abnehmbar.

Mittels der Patientenlagerungsvorrichtung 63 wird der auf der Patientenliege 67 aufliegende Patient 39 in eine einen Untersuchungsbereich aufweisende Öffnung 69 eines Magneten des Kernspintomographen 61 eingefahren, so dass darin ein 3D-Bild aufgenommen werden kann. Die Patientenlagerungsvorrichtung 63 des dargestellten Kernspintomographen 61 weist einen vom Gehäuse des Kernspintomographen 61 um ca. 30 cm beabstandeten Sockel 65 auf. Der genannte Abstand ist als freitragender Bereich der Patientenliege 67 von besonderem Vorteil beim Einsatz des Transportwagens 51, weil die Übergabe oder Übernahme der Liege 67 vereinfacht ist.

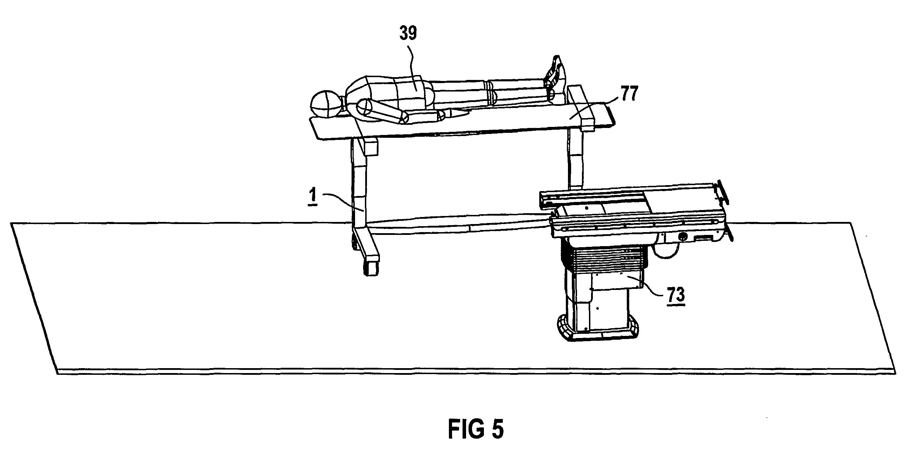

Eine weitere Anwendung des Transportwagens 1 für eine anders geartete Patientenlagerungsvorrichtung 73 mit einer zugeordneten abnehmbaren Patientenliege 77 ist in **Figur 5** dargestellt.

Die den jeweiligen Patientenlagerungsvorrichtungen 43, 63, 73 der Figuren 3 bis 5 zugeordneten Patientenliegen 47, 67, 77 sind am Fußende jeweils mit einer von oben zugänglichen und gegenüber dem zweiten Auslegerarm 13 gleichen Koppeleinrichtung 33 versehen, so dass die Schnittstelle zwischen den Patientenliegen 47, 67, 77 und dem Transportwagen 1 jeweils die gleiche ist. Der Transportwagen 1 ist somit universell einsetzbar. Mit anderen Worten: Alle Patientenliegen 47, 67, 77 des Verbunds aus unterschiedlichen Untersuchungsgeräten sind an den mindestens einen Transportwagen 1 ankoppelbar.

Das System, umfassend einen Verbund aus dem Computertomographen 41, dem Kernspintomographen 61 und einem nicht explizit dargestellten weiteren Röntgengerät (Angiographie-Gerät) (Figur 5) sowie außerdem umfassend den Transportwagen 1, stellt eine Einrichtung zur medizinischen Untersuchung eines Patienten nach der Erfindung dar. Innerhalb dieses Systems ist nur eine geringe Anzahl von Transportwägen 1 erforderlich und es ist mit hoher Wahrscheinlichkeit immer ein Transportwagen verfügbar, da diese universell einsetzbar sind.

Innerhalb des Systems ist es von der besonderem Vorteil, falls jede der Patientenliegen 47, 67, 77 derart ausgebildet ist, dass sie an jede der Patientenlagerungsvorrichtungen 43, 63, 73 ankoppelbar ist, d.h., der Koppelmechanismus jeder Patientenliege 47, 67, 77 zum Unterbau ist identisch. Bei dieser Ausgestaltung ist die Zahl notwendiger Umbettungs- oder Umlagerungsvorgänge für den Patienten 39, der mehrere Untersuchungen an verschiedenen Untersuchungsgeräten 41, 61 ("Modalitäten") durchlaufen muss, minimiert.

Ein besonderer Vorteil des Transportwagens 1 nach der Erfindung liegt in der Möglichkeit, alle bekannten Patientenliegen mit nur geringen Änderungen für den Trolleybetrieb auszulegen, ohne in die Grundkonzeption oder den Grundaufbau der jeweiligen Patientenliege oder der jeweiligen Patientenlagerungsvorrichtung eingreifen zu müssen. Die besondere Art der Anbringung der Patientenliege auf dem Transportwagen 1 gewährleistet eine maximale Patientenzugänglichkeit für das Krankenhauspersonal und für gegebenenfalls vorhandene mobile Röntgendurchleuchtungsgeräte. Der Transportwagen 1 erlaubt außerdem eine Beibehaltung der modalitätenspezifischen Form bekannter Patientenliegen an der Kopfseite, ohne dass Einschränkungen der modalitätenspezifischen Bildqualität zu befürchten wären.

Als Untersuchungs- und/oder Behandlungsgerät kann zusätzlich oder ersatzweise z.B. ein Nukleartherapiegerät oder ein Lithotripsie-Gerät vorhanden sein.

## Patentansprüche

1. Transportwagen (1) für eine Patientenliege (3), mit einem Fahrgestell (5), und mit Auslegearmen (13,15) zur Aufnahme der Patientenliege (3),
**dadurch gekennzeichnet, dass** ein erster Auslegerarm (15) der Auslegerarme zum nicht koppelnden Auflegen der Patientenliege (3) ausgebildet ist und ein zweiter Auslegerarm (13) der Auslegerarme derart zum Ankoppeln der Patientenliege (3) ausgebildet ist, dass die Patientenliege (3) im angekoppelten Zustand unterhalb des zweiten Auslegerarms (13) zu liegen kommt.

2. Transportwagen (1) nach Anspruch 1,
**dadurch gekennzeichnet, dass** der zweite Auslegerarm (13) zur hängenden Aufnahme der Patientenliege (3) ausgebildet ist.

3. Transportwagen (1) nach Anspruch 1 oder 2,
**dadurch gekennzeichnet, dass** der zweite Auslegerarm (13) ein Koppelmittel (27) zur Herstellung einer festen, lösbaren Verbindung mit der Patientenliege (3) aufweist.

4. Transportwagen (1) nach einem der Ansprüche 1 bis 3,
**dadurch gekennzeichnet, dass** der erste Auslegerarm (15) derart ausgebildet ist, dass die aufliegende Patientenliege (3) gegen seitliches Verschieben gesichert ist, vorzugsweise derart, dass die Patientenliege (3) nach oben beweglich bleibt.

5. Transportwagen (1) nach Anspruch 4,
**dadurch gekennzeichnet, dass** der erste Auslegerarm (15) eine Ausnehmung (23) für die Patientenliege (3) aufweist.

6. Transportwagen (1) nach Anspruch 1 bis 5,
**dadurch gekennzeichnet, dass** die beiden Auslegerarme (13,15) jeweils endseitig an einer Teleskopsäule (9 bzw. 11) befestigt sind.

7. Transportwagen (1) nach Anspruch 6,
**dadurch gekennzeichnet, dass** die Teleskopsäulen (9,11) außermittig auf dem Fahrgestell (5), insbesondere randseitig auf dem Fahrgestell (5), angebracht sind.

8. Transportwagen (1) nach Anspruch 6 oder 7,
**dadurch gekennzeichnet, dass** die Teleskopsäulen (9,11) sowohl gleichlaufend als auch unabhängig voneinander in der Höhe verstellbar sind.

9. Transportwagen (1) nach einem der Ansprüche 1 bis 8,
**dadurch gekennzeichnet, dass** die Auslegerarme (13,15) abnehmbar und gegeneinander austauschbar sind.

## Claims

1. Transport carriage (1) for a patient bed (3), with an undercarriage (5) and with bracket arms (13, 15) to hold the patient bed (3),
**characterised in that** a first bracket arm (15) of the bracket arms is configured to position the patient bed (3) in a non-coupling fashion and a second bracket arm (13) of the bracket arms is configured to couple the patient bed (3) such that in the coupled state the patient bed (3) comes to rest below the second bracket arm (13).

2. Transport carriage (1) according to claim 1,
**characterised in that** the second bracket arm (13) is configured to hold the patient bed (3) in a suspended fashion.

3. Transport carriage (1) according to claim 1 or 2,
**characterised in that** the second bracket arm (13) has a coupling means (27) to establish a fixed, releasable connection to the patient bed (3).

4. Transport carriage (1) according to one of claims 1 to 3,
**characterised in that** the first bracket arm (15) is configured such that the patient bed (3) resting thereon is secured against lateral displacement, preferably such that the patient bed (3) can still be moved upwards.

5. Transport carriage (1) according to claim 4,
**characterised in that** the first bracket arm (15) has a recess (23) for the patient bed (3).

6. Transport carriage (1) according to claim 1 to 5,
**characterised in that** the ends of each of the two bracket arms (13, 15) are attached to a telescopic column (9, 11).

7. Transport carriage (1) according to claim 6,
**characterised in that** the telescopic columns (9, 11) are attached eccentrically to the undercarriage (5), in particular at the edge of the undercarriage (5).

8. Transport carriage (1) according to claim 6 or 7,
**characterised in that** the telescopic columns (9, 11) are height-adjustable both in tandem and independently of each other.

9. Transport carriage (1) according to one of claims 1 to 8, **characterised in that** the bracket arms (13, 15) can be removed and interchanged with each other.

## Revendications

1. Chariot transporteur (1) pour un lit d'examen (3) comprenant un châssis (5) et des bras (13, 15) en console pour la réception du lit d'examen (3),
**caractérisé par le fait qu'**un premier des bras (15) en console est constitué de manière à recevoir le lit d'examen (3) sans couplage et qu'un deuxième des bras (13) en console est constitué de manière à se coupler au lit d'examen (3) de telle sorte que le lit d'examen (3) vienne en position couplée en-dessous du deuxième bras (13) en console.

2. Chariot transporteur (1) selon la revendication 1,
**caractérisé par le fait que** le deuxième bras (13) en console est conçu pour la réception en suspension du lit d'examen (3).

3. Chariot transporteur (1) selon les revendications 1 ou 2,
**caractérisé par le fait que** le deuxième bras (13) en console est muni d'un dispositif de couplage (27) ménageant un assemblage solide et amovible avec le lit d'examen (3).

4. Chariot transporteur (1) selon l'une quelconque des revendications 1 à 3,
**caractérisé par le fait que** le premier bras (15) en console est conçu de façon à empêcher un déplacement latéral du lit d'examen (3), de préférence de façon à ce que le lit d'examen reste mobile vers le haut.

5. Chariot transporteur (1) selon la revendication 4,
**caractérisé par le fait que** le premier bras (15) en console est muni d'un évidement (23) pour le lit d'examen (3).

6. Chariot transporteur (1) selon l'une quelconque des revendications 1 à 5,
**caractérisé par le fait que** les deux bras (13,15) en console sont chacun fixés par une extrémité à une colonne télescopique (9 ou 11).

7. Chariot transporteur (1) selon la revendication 6,
**caractérisé par le fait que** les colonnes télescopiques (9, 11) sont disposées de façon décentrée sur le châssis (5), en particulier sur le côté du châssis (5).

8. Chariot transporteur (1) selon les revendications 6 ou 7,
**caractérisé par le fait que** les colonnes télescopiques (9, 11) peuvent être réglées en hauteur tant ensemble qu'indépendamment l'une de l'autre.

9. Chariot transporteur (1) selon l'une quelconque des revendications 1 à 8,
**caractérisé par le fait que** les deux bras (13,15) en console sont démontables et interchangeables.
